# EUROPÄISCHE PATENTANMELDUNG

(11) **EP 3 770 240 A1**
(43) Veröffentlichungstag der Anmeldung: **27.01.2021**
(21) Anmeldenummer: 19187517.8
(22) Anmeldetag: 22.07.2019
(51) Int. Cl.: C11D 11/00

(54) **GESCHIRRSPÜLMITTEL MIT BLEICHKATALYSATOR UND BACILLUS GIBSONII PROTEASE**

(71) Anmelder: Henkel AG & Co. KGaA, 40589 Düsseldorf (DE)
(72) Erfinder: WEBER, Thomas, 35096 Weimar (Lahn) (DE); BASTIGKEIT, Thorsten, 42279 Wuppertal (DE); WIELAND, Susanne, 41541 Zons/Dormagen (DE); DEGERING, Christian, 40699 Erkrath (DE); MUSSMANN, Nina, 47877 Willich (DE); DOERING, Thomas, 41540 Dormagen (DE); MUELLER, Melanie, 40789 Monheim (DE); MENKE, Silke, 42781 Haan (DE); SZEMAIT, Melanie, 47167 Duisburg (DE); OTTOW, Claudia, 40878 Ratingen (DE)

(57) **Zusammenfassung**

Die Erfindung betrifft ein Geschirrspülmittel, umfassend eine Wasserstoffperoxidquelle, einen Bleichkatalysator und eine Protease, dadurch gekennzeichnet, dass die Protease eine Aminosäuresequenz umfasst, die mindestens 70% Sequenzidentität mit der in SEQ ID NO:1 angegebenen Aminosäuresequenz über deren Gesamtlänge aufweist und mindestens eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist. Ferner umfasst die Erfindung entsprechende Reinigungsverfahren, in denen ein solches Geschirrspülmittel angewendet wird, sowie entsprechende Verwendungen eines solchen Reinigungsmittels zur Entfernung von proteinhaltigen Anschmutzungen, insbesondere Eianschmutzungen, und/oder bleichbaren Anschmutzungen, insbesondere Teeanschmutzungen, von harten Oberflächen, insbesondere Geschirr.

SEQUENCE LISTING

<110>  Henkel AG & Co. KGaA

<120>  Geschirrspülmittel mit Bleichkatalysator und Bacillus gibsonii
       Protease

<130>  2019P00329EP

<160>  1

<170>  PatentIn version 3.5

<210>  1
<211>  269
<212>  PRT
<213>  Bacillus gibsonii

<400>  1

Gln Gln Thr Val Pro Trp Gly Ile Thr Arg Val Gln Ala Pro Thr Val
1               5                   10                  15

His Asn Arg Gly Ile Thr Gly Ser Gly Val Lys Val Ala Ile Leu Asp
            20                  25                  30

Thr Gly Ile Ala Gln His Ser Asp Leu Thr Ile Arg Gly Gly Ala Ser
        35                  40                  45

Phe Val Pro Gly Glu Ser Thr Thr Ala Asp Leu Asn Gly His Gly Thr
    50                  55                  60

His Val Ala Gly Thr Val Ala Ala Leu Asn Asn Ser Ile Gly Val Ile
65                  70                  75                  80

Gly Val Ala Pro Ser Ala Asp Leu Tyr Ala Val Lys Val Leu Gly Ala
                85                  90                  95

Asn Gly Arg Gly Ser Val Ser Gly Ile Ala Gln Gly Leu Glu Trp Ala
            100                 105                 110

Ala Thr Asn Asn Met His Ile Ala Asn Met Ser Leu Gly Ser Asp Ala
        115                 120                 125

Pro Ser Thr Thr Leu Glu Arg Ala Val Asn Tyr Ala Thr Ser Arg Gly
    130                 135                 140

Val Leu Val Ile Ala Ala Thr Gly Asn Asn Gly Thr Gly Ser Ile Gly
145                 150                 155                 160

Tyr Pro Ala Arg Tyr Ala Asn Ala Met Ala Val Gly Ala Thr Asp Gln
                165                 170                 175

Asn Asn Arg Arg Ala Ser Phe Ser Gln Tyr Gly Thr Gly Ile Asp Ile
            180                 185                 190

Val Ala Pro Gly Val Gly Ile Gln Ser Thr Tyr Leu Asn Asn Ser Tyr
        195                 200                 205

Ala Ser Met Pro Gly Thr Ser Met Ala Thr Pro His Val Ala Gly Val
    210                 215                 220

Ala Ala Leu Val Lys Gln Lys Asn Pro Ser Trp Asn Ala Thr Gln Ile
225                 230                 235                 240

Arg Asn His Leu Lys Asn Thr Ala Thr Asn Leu Gly Asn Ser Ser Gln
                245                 250                 255

Phe Gly Ser Gly Leu Val Asn Ala Asp Ala Ala Thr Arg
            260                 265

## Beschreibung

Die Erfindung betrifft bleichehaltige Geschirrspülmittel, umfassend mindestens eine *Bacillus gibsonii* Protease. Ebenfalls Bestandteil der Erfindung sind die entsprechenden Reinigungsverfahren sowie die Verwendung der hierin beschriebenen Mittel.

Der Einsatz von Enzymen in Wasch- und Reinigungsmitteln ist seit Jahrzehnten im Stand der Technik etabliert. Sie dienen dazu, das Leistungsspektrum der betreffenden Mittel entsprechend ihren speziellen Aktivitäten zu erweitern. Hierzu gehören insbesondere hydrolytische Enzyme wie Proteasen, Amylasen, Lipasen und Cellulasen. Die ersten drei genannten hydrolysieren Proteine, Stärke und Fette und tragen somit unmittelbar zur Schmutzentfernung bei. Cellulasen werden insbesondere wegen ihrer Gewebewirkung eingesetzt. Die am längsten etablierten und in praktisch allen modernen, leistungsfähigen Wasch- und Reinigungsmitteln enthaltenen Enzyme sind Proteasen. Sie gehören damit zu den technisch bedeutendsten Enzymen überhaupt. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet z.B. der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen. In Wasch- und Reinigungsmitteln dienen Proteasen dem Abbau proteinhaltiger Anschmutzungen auf dem Reinigungsgut.

Geschirrspülmittel stehen dem Verbraucher in einer Vielzahl von Angebotsformen zur Verfügung. Neben den traditionellen flüssigen Handgeschirrspülmitteln haben mit der Verbreitung von Haushaltsgeschirrspülmaschinen insbesondere die maschinellen Geschirrspülmittel eine große Bedeutung erlangt. Diese maschinellen Geschirrspülmittel werden dem Verbraucher typischerweise in fester Form, z.B. als Pulver oder als Tabletten, angeboten.

Eines der Hauptziele der Hersteller maschineller Reinigungsmittel ist die Verbesserung der Reinigungsleistung dieser Mittel, wobei in jüngster Zeit ein verstärktes Augenmerk auf die Reinigungsleistung bei Niedrigtemperatur-Reinigungsgängen bzw. in Reinigungsgängen mit verringertem Wasserverbrauch gelegt wird.

Proteinhaltige Anschmutzungen, insbesondere Eianschmutzungen, sowie bleichbare Anschmutzungen, insbesondere Teeanschmutzungen, stellen jedoch hartnäckige Anschmutzungen dar, die oftmals nicht zufriedenstellend entfernt werden. Moderne Geschirrspülmittel, insbesondere maschinelle Geschirrspülmittel, genügen im Hinblick auf die Beseitigung von derartigen Anschmutzungen oft nicht den gestellten Anforderungen. Es besteht daher weiterhin Bedarf an Geschirrspülmitteln und hierunter insbesondere an maschinellen Geschirrspülmitteln, die proteinhaltige und bleichbare Anschmutzungen zuverlässig entfernen, insbesondere auch bei tieferen Reinigungstemperaturen.

Diesbezüglich ist in der europäischen Patentanmeldung EP0846155 offenbart, dass durch den Zusatz von Lipase die Wirkung von Bleichmitteln auf Teeanschmutzungen erhöht wird. Aus der europäischen Patentanmeldung EP0476257 geht die Verwendung von Aminosäuren als Bleichstabilisatoren hervor.

Der vorliegenden Erfindung liegt die Aufgabe zugrunde, die Reinigungsleistung von Geschirrspülmitteln, insbesondere die Reinigungsleistung an proteinhalteigen und/oder bleichbaren Anschmutzungen, insbesondere Teeanschmutzungen, weiter zu verbessern.

Generell sind nur ausgewählte Proteasen für den Einsatz in flüssigen tensidhaltigen Zubereitungen überhaupt geeignet. Viele Proteasen zeigen in derartigen Zubereitungen keine ausreichende katalytische Leistung oder aber sie sind nicht ausreichend stabil. Für die Anwendung von Proteasen in Reinigungsmitteln ist daher eine hohe katalytische Aktivität und Stabilität unter Bedingungen, wie sie sich während eines Waschvorgangs darstellen, besonders wünschenswert.

Gegenstand der Erfindung ist daher in einem ersten Aspekt ein Geschirrspülmittel, umfassend eine Wasserstoffperoxidquelle, einen Bleichkatalysator und eine *Bacillus gibsonii* Protease, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist.

Ein weiterer Gegenstand der Erfindung ist ein Geschirrspülmittel, umfassend eine Wasserstoffperoxidquelle, einen Bleichkatalysator und eine *Bacillus gibsonii* Protease, die mindestens 70% Sequenzidentität mit der in SEQ ID NO:1 angegebenen Aminosäuresequenz über deren Gesamtlänge aufweist und die mindestens eine der der Aminosäuresubstitutionen Q12L, I43V, M122L, D127P, N154S, T156A, G160S, M211N, M211L, P212D, P212H oder A222S an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist.

Ein weiterer Gegenstand der Erfindung ist ein Verfahren zur Herstellung eines solchen Geschirspülmittels sowie ein Verfahren zur Entfernung von proteinhaltigen Anschmutzungen, insbesondere Eianschmutzungen, und/oder bleichbaren Anschmutzungen, insbesondere Teeanschmutzungen, auf harten Oberflächen, insbesondere Geschirr.

Ein weiterer Gegenstand der Erfindung ist eine Verwendung eines solchen Geschirrspülmittels zur Reinigung von harten Oberflächen, insbesondere Geschirr, insbesondere zur Entfernung von proteinhaltigen Anschmutzungen, insbesondere Eianschmutzungen, und/oder bleichbaren Anschmutzungen, insbesondere Teeanschmutzungen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichts-%. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden. "Mindestens eine", wie hierin verwendet, bedeutet eine oder mehrere, d.h. 1, 2, 3, 4, 5, 6, 7, 8, 9, 10, 11, 12, 13, 14 oder mehr. Der Begriff "Geschirrspülmittel", wie hierin verwendet, ist gleichbedeutend mit dem Begriff "Reinigungsmittel" oder "Mittel" und bezeichnet eine Zusammensetzung zum Reinigen von harten Oberflächen, insbesondere Geschirr, wie in der Beschreibung erläutert. "Etwa", "ca." oder "ungefähr", wie hierin in Bezug auf einen Zahlenwert verwendet, beziehen sich auf den entsprechenden Zahlenwert ± 10%, vorzugsweise ± 5%.

Die vorliegende Erfindung basiert auf der überraschenden Erkenntnis der Erfinder, dass Geschirrspülmittel, die Proteasen umfassen, die eine Aminosäuresequenz umfassen, die mindestens 70% Sequenzidentität mit der in SEQ ID NO:1 angegebenen Aminosäuresequenz über deren Gesamtlänge aufweist und mindestens eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist, nicht nur eine verbesserte Reinigungsleistung an proteinhaltigen Anschmutzungen, insbesondere Eianschmutzungen, aufweisen, sondern auch in Kombination mit einem Bleichkatalysator, eine sehr gute Bleichleistung aufweisen und folglich eine sehr gute Reinigungsleistung an bleichbaren Anschmutzungen, insbesondere Teeanschmutzungen, zeigen.

Das ist insbesondere insoweit überraschend, als bisher keine solche *Bacillus gibsonii* Protease mit einer verbesserten Reinigungsleistung in Geschirrspülmitteln, insbesondere nicht im Hinblick auf proteinhaltige und bleichbare Anschmutzungen, in Verbindung gebracht wurde. Insbesondere die sehr gute Reinigungsleistung an bleichbaren Anschmutzungen ist bemerkenswert, da Proteasen im Allgemeinen empfindlich auf Bleichmittel in Wasch- und Reinigungsmittel reagieren und in Gegenwart von Bleichmitteln nicht gut funktionieren. Die erfindungsgemäßen Proteasen scheinen nicht nur weniger empfindlich gegenüber Bleichmitteln im Reinigungsmittel zu sein und somit eine generell verbesserte Reinigungsleistung an proteinhaltigen Anschmutzungen zu zeigen, sondern in bevorzugten Ausgestaltungen erfindungsgemäßer Mittel liegt zudem ein Synergismus zwischen der entsprechenden Protease und dem Bleichkatalysator vor hinsichtlich der Reinigungsleistung, insbesondere hinsichtlich der Reinigungsleistung an Teeanschmutzungen.

Weitere bevorzugte Ausgestaltungen erfindungsgemäßer Mittel zeigen solche vorteilhaften Reinigungsleistungen auch bei niedrigen Temperaturen und/oder bei kurzen Spülgängen. Eine niedrige Temperatur im Sinne der Erfindung liegt vorzugsweise zwischen 10 und 50°C, vorzugsweise zwischen 15 und 45°C und besonders bevorzugt zwischen 20 und 40°C vor. Ein kurzer Spülgang dauert vorzugsweise maximal 60 Minuten, 45 Minuten oder lediglich maximal 30 Minuten. Weitere bevorzugte Ausgestaltungen erfindungsgemäßer Mittel zeigen ferner eine verbesserte Klarspülleistung.

Die Reinigungsleistung beschreibt das Vermögen eines Geschirrspülmittels, insbesondere eines maschinellen Geschirrspülmittels, eine vorhandene Anschmutzung teilweise oder vollständig zu entfernen. Im Rahmen der Erfindung weist sowohl das Geschirrspülmittel, welches die Protease umfasst bzw. die durch dieses Mittel gebildete Reinigungsflotte, als auch die Protease selbst eine jeweilige Reinigungsleistung auf. Die Reinigungsleistung des Enzyms trägt somit zur Reinigungsleistung des Mittels bzw. der durch das Mittel gebildeten Reinigungsflotte bei.

Unter Reinigungsflotte wird diejenige das Geschirrspülmittel enthaltende Gebrauchslösung verstanden, die auf die harten Oberflächen einwirkt und damit mit den auf den harten Oberflächen vorhandenen Anschmutzungen in Kontakt kommt. Üblicherweise entsteht die Reinigungsflotte, wenn der Reinigungsvorgang beginnt und das Geschirrspülmittel z.B. in einer Spülmaschine oder in einem anderen geeigneten Behältnis mit Wasser verdünnt wird.

Die Reinigungsleistung kann in einem System bestimmt werden, das ein maschinelles Geschirrspülmittel in einer Dosierung wie hierin angegeben sowie die Protease enthält, wobei die zu vergleichenden Proteasen konzentrationsgleich (bezogen auf aktives Protein) eingesetzt sind und die Reinigungsleistung gegenüber einer Anschmutzung von Tee, Fleisch, Spaghetti, Ei und/oder Creme Brûlée gemäß IKW Methode in einer Miele GSL (Programm 45°C, 21°dH) bestimmt wird. Die Konzentration der Protease in dem für dieses Waschsystem bestimmten Mittels beträgt 0,001 bis 0,1 Gew.-%, vorzugsweise 0,01 bis 0,06 Gew.-%, bezogen auf aktives Protein.

Ein Referenzmittel für ein solches Waschsystem kann wie folgt zusammengesetzt sein:

| | Gew.-% |
|---|---|
| Citrat, Na-Salz | 15-20 |
| Phosphonat (z.B. HEDP) Sofern regulatorisch zulässig | 0-7,5 (2,5-7,5) |
| MGDA, Na-Salz | 0-25 |
| Disilicat, Na-Salz | 5-35 |
| Soda | 10-25 |
| Silberschutz | 0,0 - 1,0 |
| Percarbonat, Na-Salz | 10-15 |
| Bleichkatalysator (bevorzugt Mn-basiert) | 0,02-0,5 |
| Bleichaktivator (z.B. TAED) | 1-3 |
| Nichtionische(s) Tensid(e), z.B. Fettalkoholalkoxylat, bevorzugt 20-40 EO, ggf. endcapped | 2,5-10 |
| Polycarboxylat | 4 - 10 |
| Kationisches Copolymer | 0 - 0,75 |
| Disintegrant - (z.B. Crosslinked PVP) | 0 -1,5 |
| Protease-Zubereitung (tq) | 0-5 |
| Amylase-Zubereitung (tq) | 0-3 |
| Parfüm | 0,05-0,25 |
| Farbstofflösung | 0,0-1 |
| Zink-Salz | 0,1-0,3 |
| Natriumsulfat | 0,0 - 10 |
| Wasser | 0,0-1,5 |
| pH-Stellmittel (z.B. Citronensäure) | 0-1,5 |
| Prozesshilfsmittel | 0-5 |

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also z.B. die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Verfahren zur Bestimmung der Proteaseaktivität sind dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Die Proteaseaktivität wird üblicherweise in Protease-Einheiten (PE) angegeben. Geeignete Proteaseaktivitäten betragen z.B. 2,25, 5 oder 10 PE pro ml Spülflotte oder Spülvorgang. Die Proteaseaktivität ist jedoch nicht gleich Null.

Die Proteinkonzentration kann mit Hilfe bekannter Methoden, z.B. dem BCA-Verfahren (Bicinchoninsäure; 2,2'-Bichinolyl-4,4'-dicarbonsäure) oder dem Biuret-Verfahren (Gornall et al., 1948, J. Biol. Chem., 177:751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (Bender et al., 1966, J. Am. Chem. Soc. 88(24):5890-5913) erfolgen.

Die erfindungsgemäß eingesetzten Proteasen weisen enzymatische Aktivität auf, d.h., sie sind zur Hydrolyse von Peptiden und Proteinen befähigt, insbesondere in Wasch- oder Reinigungsmittel. Eine erfindungsgemäß eingesetzte Protease ist daher ein Enzym, welches die Hydrolyse von Amid/Peptidbindungen in Protein/Peptid-Substraten katalysiert und dadurch in der Lage ist, Proteine oder Peptide zu spalten. Ferner handelt es sich bei einer erfindungsgemäß eingesetzten Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Protease Moleküle durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus gibsonii* oder *Bacillus subtilis,* die die erfindungsgemäßen Proteasen exprimieren, oder Zellkulturüberstände solcher Zellen enthalten.

Überraschenderweise sind es genau solche Proteasen, die eine Aminosäuresequenz umfassen, die mindestens 70% Sequenzidentität mit der in SEQ ID NO:1 angegebenen Aminosäuresequenz über deren Gesamtlänge aufweist und eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist, die in erfindungsgemäßen Geschirrspülmitteln zum einen eine Verbesserung der Reinigungsleistung an proteinhaltigen Anschmutzungen, insbesondere Eianschmutzungen, und zum anderen im Zusammenwirken mit dem Bleichkatalysator in einem erfindungsgemäßen Mittel eine verbesserte Reinigungsleistung an bleichbaren Anschmutzungen, insbesondere Teeanschmutzungen, bewirken. Vorzugsweise liegt diesbezüglich ein synergistisches Zusammenwirken vor.

Die erfindungsgemäß eingesetzte *Bacillus gibsonii* Protease enthält in verschiedenen bevorzugten Ausführungsformen mindestens eine Aminosäuresubstitution, die aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist.

In weiter bevorzugten Ausführungsformen enthält die erfindungsgemäß eingesetzte Protease eine der folgenden Aminosäuresubstitutionsvarianten: (i) I43V; (ii) M122L, N154S und T156A; (iii) M211N und P212D; (iv) M211L und P212D; (v) G160S; (vi) D127P, M211L und P212D; (vii) P212H; oder (viii) Q12L, M122L und A222S, wobei die Nummerierung jeweils bezogen ist auf die Nummerierung gemäß SEQ ID NO:1.

In weiteren Ausführungsformen der Erfindung umfasst die erfindungsgemäß eingesetzte Protease eine Aminosäuresequenz, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die an mindestens einer der Positionen, die den Positionen 12, 43, 122, 127, 154, 156, 160, 211, 212 oder 222 in der Zählung gemäß SEQ ID NO:1 entsprechen, eine oder mehrere der Aminosäuresubstitutionen 12L, 43V, 122L, 127P, 154S, 156A, 160S, 211 N, 211L, 212D, 212H oder 222S aufweist.

Im Zusammenhang mit der vorliegenden Erfindung bedeutet das Merkmal, dass eine Protease die angegebenen Substitutionen aufweist, dass sie mindestens eine der entsprechenden Aminosäuren an den entsprechenden Positionen enthält, d.h. nicht alle der 10 Positionen anderweitig mutiert oder, z.B. durch Fragmentierung der Protease, deletiert sind.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) "Basic local alignment search tool", J. Mol. Biol. 215:403-410 und Altschul et al. (1997) "Gapped BLAST and PSI-BLAST: a new generation of protein database search programs", Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden z.B. die Clustal-Serie (vgl. z.B. Chenna et al. (2003) "Multiple sequence alignment with the Clustal series of programs", Nucleic Acid Res. 31:3497-3500), T-Coffee (vgl. z.B. Notredame et al. (2000) "T-Coffee: A novel method for multiple sequence alignments", J. Mol. Biol. 302:205-217) oder Programme, die auf diesen Programmen bzw. Algorithmen basieren. Ferner möglich sind Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, d.h. dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäureaustausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz. Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

Ein weiterer Gegenstand der vorliegenden Erfindung ist ein Reinigungsmittel mit einer Protease, dadurch gekennzeichnet, dass die Protease aus einer erfindungsgemäßen Protease als Ausgangsmolekül durch ein- oder mehrfache konservative Aminosäuresubstitution erhältlich ist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z.B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Ein weiterer Gegenstand der vorliegenden Erfindung ist ein Reinigungsmittel mit einer Protease, dadurch gekennzeichnet, dass die Protease aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 261, 262, 263, 264, 265, 266, 267, 268 oder 269 zusammenhängenden Aminosäuren mit dem Ausgangsmolekül übereinstimmt.

So ist es z.B. möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese z.B. auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms. Auch Substitutionen können vorteilhafte Wirkungen zeigen. Sowohl einzelne wie auch mehrere zusammenhängende Aminosäuren können gegen andere Aminosäuren ausgetauscht werden.

Eine erfindungsgemäße Protease kann zusätzlich stabilisiert sein, insbesondere durch eine oder mehrere Mutationen, z.B. Substitutionen, oder durch Kopplung an ein Polymer. Denn eine Erhöhung der Stabilität bei der Lagerung und/oder während des Einsatzes, z.B. beim Reinigungsprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Weitere Möglichkeiten der Stabilisierung sind z.B.:
- Veränderung der Bindung von Metallionen, insbesondere der Calcium-Bindungsstellen, z.B. durch Austauschen einer oder mehrerer der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss denaturierender Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Protease wie vorstehend beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert. Unter Derivaten werden im Sinne der vorliegenden Anmeldung demnach solche Proteine verstanden, deren reine Aminosäurekette chemisch modifiziert worden ist. Solche Derivatisierungen können z.B. *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das z.B. über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können z.B. die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann z.B. für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit z.B. dessen Hautverträglichkeit zu erhöhen. Beispielsweise können Kopplungen mit makromolekularen Verbindungen, z.B. Polyethylenglykol, das Protein hinsichtlich der Stabilität und/oder Hautverträglichkeit verbessern. Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, z.B. aus der Kultur der produzierenden Mikroorganismen. Ein Protein kann auch, z.B. zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann z.B. über entsprechende Begleitstoffe gesteuert werden.

Zahlreiche Proteasen und insbesondere Subtilisine werden als sogenannte Präproteine, also zusammen mit einem Propeptid und einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins. Das Signalpeptid wird unter natürlichen Bedingungen durch eine Signalpeptidase von der übrigen Protease abgespalten. Anschließend erfolgt die durch das Propeptid unterstützte korrekte endgültige Faltung der Protease. Die Protease ist dann in ihrer aktiven Form und spaltet das Propeptid selbst ab. Nach der Abspaltung des Propeptids übt die dann reife (mature) Protease, insbesondere Subtilisin, ihre katalytische Aktivität ohne die ursprünglich vorhandenen N-terminalen Aminosäuren aus. Für technische Anwendungen allgemein und insbesondere im Rahmen der Erfindung sind die reifen (maturen) Proteasen, d.h. die nach ihrer Herstellung prozessierten Enzyme, gegenüber den Präproteinen bevorzugt. Die Proteasen können ferner von den sie produzierenden Zellen nach der Herstellung der Polypeptidkette modifiziert werden, z.B. durch Anknüpfung von Zuckermolekülen, Formylierungen, Aminierungen, usw. Solche Modifikationen sind posttranslationale Modifikationen und können, müssen jedoch nicht einen Einfluss auf die Funktion der Protease ausüben.

"Variante", wie hierin verwendet, bezieht sich auf natürliche oder artifiziell erzeugte Variationen einer nativen Protease, die eine gegenüber der Referenzform abgewandelte Aminosäuresequenz aufweist. Zusätzlich zu den vorstehend erläuterten Aminosäureveränderungen können erfindungsgemäße Proteasen weitere Aminosäureveränderungen, insbesondere Aminosäuresubstitutionen, -insertionen oder -deletionen, aufweisen. Solche Proteasen sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden. Das Ziel ist es, in die bekannten Moleküle gezielte Mutationen wie Substitutionen, Insertionen oder Deletionen einzuführen, um z.B. die Reinigungsleistung von Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität des Enzyms erhöht und dadurch seine Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease kann daher im Rahmen der Erfindung zusätzlich weiterentwickelt sein, z.B. hinsichtlich ihrer Stabilität gegenüber Tensiden und/oder Bleichmitteln und/oder anderen Komponenten.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P14H die Substitution von Prolin an Position 14 durch Histidin, P14HT die Insertion von Threonin nach der Aminosäure Histidin an Position 14 und P14* oder ΔP14 die Deletion von Prolin an Position 14. Diese Nomenklatur ist dem Fachmann auf dem Gebiet der Enzymtechnologie bekannt.

Die Aminosäurepositionen werden durch ein Alignment der Aminosäuresequenz einer erfindungsgemäß eingesetzten Protease mit der Aminosäuresequenz der Protease aus *Bacillus gibsonii*, wie sie in SEQ ID NO:1 angegeben ist, definiert. Weiterhin richtet sich die Zuordnung der Positionen nach dem reifen (maturen) Protein. Diese Zuordnung ist insbesondere auch dann anzuwenden, wenn die Aminosäuresequenz einer erfindungsgemäß eingesetzten Protease eine höhere Zahl von Aminosäureresten umfasst als die Protease aus *Bacillus gibsonii* gemäß SEQ ID NO:1. Ausgehend von den genannten Positionen in der Aminosäuresequenz der Protease aus *Bacillus gibsonii* sind die Veränderungspositionen in einer erfindungsgemäß eingesetzten Protease diejenigen, die eben diesen Positionen in einem Alignment zugeordnet sind.

In einer weiteren Ausführungsform der Erfindung ist die erfindungsgemäß eingesetzte Protease dadurch gekennzeichnet, dass ihre Reinigungsleistung gegenüber derjenigen einer Protease, die eine Aminosäuresequenz umfasst, die der in SEQ ID NO:1 angegebenen Aminosäuresequenz entspricht, nicht signifikant verringert ist, d.h. mindestens 80% der Referenzwaschleistung besitzt, vorzugsweise mindestens 100%, weiter bevorzugt mindestens 110% oder mehr. Die Reinigungsleistung kann wie weiter oben beschrieben bestimmt werden.

Ein erfindungsgemäßes Geschirrspülmittel enthält die Protease zunehmend bevorzugt in einer Menge von 1 x 10⁻⁸ bis 10 Gew.-%, 0,00001 bis 2 Gew.-%, 0,001 bis 1 Gew.-%, 0,007 bis 0,8 Gew.-%, 0,025 bis 0,5 Gew.-% und besonders bevorzugt von 0,04 bis 0,38 Gew.-%, bezogen auf aktives Protein.

Ein erfindungsgemäßes Geschirrspülmittel umfasst ferner einen Bleichaktivator. Bei diesen Stoffen handelt es sich vorzugsweise um bleichverstärkende Übergangsmetallsalze bzw. Übergangsmetallkomplexe wie z.B. Mn-, Fe-, Co-, Ru- oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Mit besonderem Vorzug werden Komplexe des Mangans in der Oxidationsstufe II, III, IV oder IV eingesetzt, die vorzugsweise einen oder mehrere makrocyclische(n) Ligand(en) mit den Donorfunktionen N, NR, PR, O und/oder S enthalten. Vorzugsweise werden Liganden eingesetzt, die Stickstoff-Donorfunktionen aufweisen. Dabei ist es besonders bevorzugt, Bleichkatalysator(en) in den erfindungsgemäßen Mitteln einzusetzen, welche als makromolekularen Liganden 1,4,7-Trimethyl-1,4,7-triazacyclononan (Me-TACN), 1,4,7-Triazacyclononan (TACN), 1,5,9-Trimethyl-1,5,9-triazacyclododecan (Me-TACD), 2-Methyl-1,4,7-trimethyl-1,4,7-triazacyclononan (Me/Me-TACN) und/oder 2-Methyl-1,4,7-triazacyclononan (Me/TACN) enthalten. Geeignete Mangankomplexe sind z.B. [Mn^{III}₂(µ-O)₁(µ-OAc)₂(TACN)₂](ClO₄)₂, [Mn^{III}Mn^{IV}(µ-O)₂(µ-OAc)₁(TACN)₂](BPh₄)₂, [Mn^{IV}₄(µ-O)₆(TACN)₄](ClO₄)₄, [Mn^{III}₂(µ-O)₁(µ-OAc)₂(Me-TACN)₂](ClO₄)₂, [Mn^{III}Mn^{IV}(µ-O)₁(µ-OAc)₂(Me-TACN)₂](ClO₄)₃, [Mn^{IV}₂(µ-O)₃(Me-TACN)₂](PF₆)₂ und [Mn^{IV}₂(µ-O)₃(Me/Me-TACN)₂](PF₆)₂ (OAc = OC(O)CH₃).

Geschirrspülmittel, insbesondere maschinelle Geschirrspülmittel, dadurch gekennzeichnet, dass sie einen Bleichkatalysator ausgewählt aus der Gruppe der bleichverstärkenden Übergangsmetallsalze und Übergangsmetallkomplexe, vorzugsweise aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN) enthalten, werden erfindungsgemäß bevorzugt, da durch die vorgenannten Bleichkatalysatoren insbesondere das Reinigungsergebnis signifikant verbessert werden kann.

Die vorgenannten bleichverstärkenden Übergangsmetallkomplexe, insbesondere mit den Zentralatomen Mn und Co werden vorzugsweise in einer Menge bis zu 5 Gew.-%, insbesondere 0,0025 bis 1 Gew.-% und besonders bevorzugt 0,01 bis 0,30 Gew.-%, jeweils bezogen auf das Gesamtgewicht der bleichkatalysatorhaltigen Mittel, eingesetzt. In speziellen Fällen kann jedoch auch mehr Bleichkatalysator eingesetzt werden.

Ein erfindungsgemäßes Geschirrspülmittel umfasst ferner eine Wasserstoffperoxidquelle. Hierbei handelt es sich um Verbindungen, die in Wasser H₂O₂ liefern bzw. liefern können. Bei der Wasserstoffperoxidquelle handelt es sich vorzugsweise um ein Bleichmittel, wobei Sauerstoffbleichmittel erfindungsgemäß bevorzugt werden.

Unter den als Bleichmittel dienenden, in Wasser H₂O₂ liefernden Verbindungen haben das Natriumpercarbonat, das Natriumperborattetrahydrat und das Natriumperboratmonohydrat besondere Bedeutung. Weitere brauchbare Bleichmittel sind z.B. Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure.

Weiterhin können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden.

Vorzugsweise ist die Wasserstoffperoxidquelle in einer Menge von 2 bis 30 Gew.-% und zunehmend bevorzugt 4 bis 25 Gew.-%, 5 bis 20 Gew.-% und besonders bevorzugt 6 bis 15 Gew.-% in dem erfindungsgemäßen Geschirrspülmittel enthalten, jeweils bezogen auf das Gesamtgewicht des Geschirrspülmittels. Bevorzugte Geschirrspülmittel sind ferner dadurch gekennzeichnet, dass das Geschirrspülmittel, jeweils bezogen auf das Gesamtgewicht des Geschirrspülmittels, 2 bis 20 Gew.-%, vorzugsweise 3 bis 18 Gew.-% und insbesondere 4 bis 15 Gew.-% Natriumpercarbonat enthält.

Besonders bevorzugte Ausführungsformen erfindungsgemäßer Geschirrspülmittel sind folglich dadurch gekennzeichnet, dass der Bleichkatalysator ausgewählt ist aus der Gruppe der bleichverstärkenden Übergangsmetallsalze und Übergangsmetallkomplexe, vorzugsweise aus der Gruppe der Komplexe des Mangans mit 1,4,7-trimethyl-1,4,7-triazacyclononan (Me-TACN) oder 1,2,4,7-tetramethyl-1,4,7-triazacyclononan (Me/Me-TACN), und/oder die Wasserstoffperoxidquelle Natriumpercarbonat, Natriumperborattetrahydrat oder Natriumperboratmonohydrat oder eine Kombination hiervon ist. Ganz besonders bevorzugt ist der Bleichkatalysator ein Komplex des Mangans mit 1,4,7-trimethyl-1,4,7-triazacyclononan (Me-TACN), insbesondere [Mn^{IV}₂(µ-O)₃(Me-TACN)₂](PF₆)₂, oder 1,2, 4,7-tetramethyl-1,4,7-triazacyclononan (Me/Me-TACN) oder eine Mischung hieraus, und die Wasserstoffperoxidquelle Natriumpercarbonat. In den genannten Kombinationen liegen der Bleichkatalysator und die Wasserstoffperoxidquelle vorzugsweise in den vorstehend jeweils genannten Mengen vor.

Erfindungsgemäße Geschirrspülmittel, insbesondere maschinelle Geschirrspülmittel, können ferner Bleichaktivatoren enthalten, z.B. um beim Reinigen bei Temperaturen von 60°C und darunter eine verbesserte Bleichwirkung zu erreichen. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder ggf. substituierte Perbenzoesäure ergeben, eingesetzt werden. Geeignet sind Substanzen, die O- und/oder N-Acylgruppen der genannten C-Atomzahl und/oder ggf. substituierte Benzoylgruppen tragen. Bevorzugt werden mehrfach acylierte Alkylendiamine, wobei sich Tetraacetylethylendiamin (TAED) als besonders geeignet erwiesen hat.

Diese Bleichaktivatoren, insbesondere TAED, werden vorzugsweise in Mengen von 0,1 bis 10 Gew.-%, insbesondere 0,1 bis 8 Gew.-%, besonders 2 bis 8 Gew.-% und besonders bevorzugt 2 bis 6 Gew.-%, jeweils bezogen auf das Gesamtgewicht der bleichaktivatorhaltigen Mittel, eingesetzt.

In einer bevorzugten Ausführungsform der Erfindung handelt es sich bei einem erfindungsgemäßen Geschirrspülmittel um ein maschinelles Geschirrspülmittel. Als maschinelle Geschirrspülmittel werden nach Maßgabe dieser Anmeldung Zusammensetzungen bezeichnet, die zur Reinigung verschmutzten Geschirrs in einem maschinellen Geschirrspülverfahren eingesetzt werden können. Damit unterscheiden sich die erfindungsgemäßen maschinellen Geschirrspülmittel z.B. von den maschinellen Klarspülmitteln, die stets in Kombination mit maschinellen Geschirrspülmitteln eingesetzt werden und keine eigene Reinigungswirkung entfalten.

An maschinell gespültes Geschirr werden häufig höhere Anforderungen gestellt als an manuell gespültes Geschirr. So soll das Geschirr nach der maschinellen Reinigung nicht nur frei von Speiseresten sein, sondern z.B. auch keine weißlichen, auf Wasserhärte oder anderen mineralischen Salzen beruhenden Flecken aufweisen, die mangels Netzmittel aus eingetrockneten Wassertropfen stammen. Moderne maschinelle Geschirrspülmittel erfüllen diese Anforderungen durch die Integration reinigender und/oder pflegender und/oder wasserenthärtender und/oder klarspülaktiver Wirkstoffe und sind dem Verbraucher z.B. als "2in1"- oder "3in1" Geschirrspülmittel bekannt. Als für den Reinigungs- wie für den Klarspülerfolg wesentlichen Bestandteil enthalten die maschinellen Geschirrspülmittel Gerüststoffe. Diese Gerüststoffe erhöhen zum einen die Alkalität der Reinigungsflotte, wobei mit steigender Alkalität Fette und Öle emulgiert und verseift werden, und vermindern zum anderen durch Komplexierung der in der wässrigen Flotte enthaltenen Calciumionen die Wasserhärte der Reinigungsflotte.

Das erfindungsgemäße Geschirrspülmittel kann ferner mindestens einen weiteren Inhaltsstoff, der ausgewählt ist aus der Gruppe bestehend aus Tenside, Builder (Gerüststoffe), (anionisches) Polymere, Glaskorrosionsinhibitoren, Korrosionsinhibitoren sowie Kombinationen hiervon, umfassen. Ferner können sie wassermischbare organische Lösungsmittel, weitere Enzyme, Enzymstabilisatoren, Sequestrierungsmittel, Elektrolyte, pH-Regulatoren und/oder weitere Hilfsstoffe wie optische Aufheller, Vergrauungsinhibitoren, Farbübertragungsinhibitoren, Schaumregulatoren sowie Farb- und Duftstoffe sowie Kombinationen hiervon enthalten. In einer weiteren Ausführungsform der Erfindung ist ein erfindungsgemäßes Geschirrspülmittel phosphatfrei. Erfindungsgemäße phosphatfreie Geschirrspülmittel sind insbesondere unter Umweltaspekten vorteilhaft.

Vorzugsweise werden die Inhaltsstoffe der Mittel aufeinander abgestimmt. Bevorzugt sind Synergien hinsichtlich der Reinigungsleistung und/oder der Klarspülleistung und/oder der Belagsinhibierung. Besonders bevorzugt sind Synergien, die in einem Temperaturbereich zwischen 10 und 100°C vorhanden sind, insbesondere in einem Temperaturbereich von 15 bis 100°C, 20 bis 70°C, 20 bis 60°C, 20 bis 50°C, 20 bis 40°C, 15 bis 30°C und 15 bis 25°C.

Erfindungsgemäße Geschirrspülmittel, insbesondere maschinelle Geschirrspülmittel, können ein Tensid oder mehrere Tenside enthalten, wobei insbesondere anionische Tenside, nichtionische Tenside und deren Gemische in Frage kommen.

Unter den anionischen Tensiden bevorzugt sind solche, die mindestens eine Sulfat- oder Sulfonat-Gruppe aufweisen. Das anionische Tensid mit mindestens einer Sulfat- oder Sulfonat-Gruppe ist vorzugsweise ausgewählt aus Fettalkoholsulfaten, Alkansulfonaten und Alkylbenzolsulfonaten. Bevorzugt sind hierbei C₁₂₋₁₈-Fettatkohot-Sutfate (FAS), z.B. Sulfopon K 35 (Cognis, Deutschland), sekundäre C₁₃₋₁₇-Alkansulfonate (SAS), z.B. Hostapur SAS 93 (Clariant, Deutschland), sowie lineare C₈₋₁₈-Alkylbenzolsulfonate, insbesondere Dodecylbenzolsulfonat (LAS). Erfindungsgemäß umfassen die Begriffe "Sulfat" und "Sulfonat" neben betreffenden anionischen Verbindungen, die in Form von Salzen vorliegen, auch die freien Säuren, also die entsprechenden Alkylschwefelsäuren bzw. Alkylsulfonsäuren. Vorzugsweise ist das anionische Tensid mit mindestens einer Sulfat- oder Sulfonat-Gruppe in erfindungsgemäßen Geschirrspülmitteln in einer Menge von 0,1 bis 20 Gew.-%, besonders bevorzugt von 0,5 bis 15 Gew.-%, insbesondere von 2,5 bis 10 Gew.-%, enthalten.

Ein bevorzugter Bestandteil der erfindungsgemäßen Reinigungsmittel sind die nichtionischen Tenside, wobei nichtionische Tenside der allgemeinen Formel R¹-CH(OH)CH₂O-(AO)_{w}-(A'O)ₓ-(A"O)_{y}-(A"'O)_{z}-R², in der R¹ für einen geradkettigen oder verzweigten, gesättigten oder ein- bzw. mehrfach ungesättigten C₆₋₂₄-Alkyl- oder -Alkenylrest steht; R² für einen linearen oder verzweigten Kohlenwasserstoffrest mit 2 bis 26 Kohlenstoffatomen steht; A, A', A"' und A"" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen, w, x, y und z für Werte zwischen 0,5 und 120 stehen, wobei x, y und/oder z auch 0 sein können, bevorzugt sind.

Durch den Zusatz der vorgenannten nichtionischen Tenside der allgemeinen Formel R¹-CH(OH)CH₂O-(AO)_{w}-(A'O)ₓ-(A"O)_{y}-(A"'O)_{z}-R², nachfolgend auch als "Hydroxymischether" bezeichnet, kann überraschenderweise die Reinigungsleistung erfindungsgemäßer enzymhaltiger Zubereitungen deutlich verbessert werden und zwar sowohl im Vergleich zu tensidfreien System wie auch im Vergleich zu Systemen, die alternative nichtionischen Tenside, z.B. aus der Gruppe der polyalkoxylierten Fettalkohole enthalten.

Durch den Einsatz dieser nichtionischen Tenside mit einer oder mehreren freien Hydroxylgruppe(n) an einem oder beiden endständigen Alkylreste kann die Stabilität der in den erfindungsgemäßen Reinigungsmittelzubereitungen enthaltenen Enzyme deutlich verbessert werden.

Bevorzugt werden insbesondere solche endgruppenverschlossene poly(oxyalkylierten) Niotenside, die, gemäß der Formel R¹O[CH₂CH₂O]ₓCH₂CH(OH)R², neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei x für Werte zwischen 1 und 90, vorzugsweise für Werte zwischen 30 und 80 und insbesondere für Werte zwischen 30 und 60 steht.

Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht. Zur Gruppe dieser nichtionischen Tenside zählen z.B. die C₂₋₂₆ Fettalkohol-(PO)₁-(EO)₁₅₋₄₀-2-hydroxyalkylether, insbesondere auch die C₈₋₁₀ Fettalkohol-(PO)₁-(EO)₂₂-2-hydroxydecylether.

Besonders bevorzugt werden weiterhin solche endgruppenverschlossene poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für-CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R₃)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der obenstehenden Formel unterschiedlich sein, falls x ≥ 2 ist. Hierdurch kann die Alkylenoxid-Einheit in der eckigen Klammer variiert werden. Steht x z.B. für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, z.B. (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und z.B. eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der oben stehenden Formel weisen Werte von k = 1 und j = 1 auf, so dass sich die vorstehende Formel zu R¹O[CH₂CH(R³)O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Als besonders wirkungsvoll haben sich schließlich die nichtionischen Tenside der allgemeine Formel R¹-CH(OH)CH₂O-(AO)_{w}-R² erwiesen, in der R¹ für einen geradkettigen oder verzweigten, gesättigten oder ein- bzw. mehrfach ungesättigten C₆₋₂₄-Alkyl- oder -Alkenylrest steht; R² für einen linearen oder verzweigten Kohlenwasserstoffrest mit 2 bis 26 Kohlenstoffatomen steht; A für einen Rest aus der Gruppe -CH₂CH₂, -CH₂CH₂-CH₂, -CH₂-CH(CH₃) steht, und w für Werte zwischen 1 und 120, vorzugsweise 10 bis 80, insbesondere 20 bis 40 steht. Zur Gruppe dieser nichtionischen Tenside zählen z.B. die C₄₋₂₂ Fettalkohol-(EO)₁₀₋₈₀-2-hydroxyalkylether, insbesondere auch die C₈₋₁₂ Fettalkohol-(EO)₂₂-2-hydroxydecylether und die C₄₋₂₂ Fettalkohol-(EO)₄₀₋₈₀-2-hydroxyalkylether.

Bevorzugte Reinigungsmittel sind dadurch gekennzeichnet, dass das Reinigungsmittel mindestens ein nichtionisches Tensid, vorzugsweise ein nichtionisches Tensid aus der Gruppe der Hydroxymischether, enthält, wobei der Gewichtsanteil des nichtionischen Tensids am Gesamtgewicht des Reinigungsmittels vorzugsweise 0,2 bis 10 Gew.-%, bevorzugt 0,4 bis 7,0 Gew.-% und insbesondere 0,6 bis 6,0 Gew.-% beträgt.

Bevorzugte erfindungsgemäße Mittel zur Verwendung in maschinellen Geschirrspülverfahren können neben den zuvor beschriebenen nichtionischen Tensiden weitere Tenside, insbesondere amphotere Tenside, enthalten. Der Anteil anionischer Tenside am Gesamtgewicht dieser Mittel ist jedoch vorzugsweise begrenzt. So sind bevorzugte maschinelle Geschirrspülmittel dadurch gekennzeichnet, dass diese bezogen auf ihr Gesamtgewicht weniger als 5,0 Gew.-%, vorzugsweise weniger als 3,0 Gew.-%, besonders bevorzugt weniger als 2,0 Gew.-% Aniontensid enthalten. Auf den Einsatz von anionischen Tensiden in größerer Menge wird dabei insbesondere zur Vermeidung einer übermäßigen Schaumentwicklung verzichtet.

Ein weiterer bevorzugter Bestandteil erfindungsgemäßer Mittel sind Komplexbildner. Besonders bevorzugte Komplexbildner sind die Phosphonate, sofern ihr Einsatz regulatorisch zulässig ist. Die komplexbildenden Phosphonate umfassen neben der 1-Hydroxyethan-1,1-diphosphonsäure eine Reihe unterschiedlicher Verbindungen wie z.B. Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Ein im Rahmen dieser Anmeldung bevorzugtes Mittel enthält ein oder mehrere Phosphonat(e) aus der Gruppe Aminotrimethylenphosphonsäure (ATMP) und/oder deren Salze; Ethylendiamintetra(methylenphosphonsäure) (EDTMP) und/oder deren Salze; Diethylentriaminpenta(methylenphosphonsäure) (DTPMP) und/oder deren Salze; 1-Hydroxyethan-1,1-diphosphonsäure (HEDP) und/oder deren Salze; 2-Phosphonobutan-1,2,4-tricarbonsäure (PBTC) und/oder deren Salze; Hexamethylendiamintetra(methylenphosphonsäure) (HDTMP) und/oder deren Salze; Nitrilotri(methylenphosphonsäure) (NTMP) und/oder deren Salze.

Besonders bevorzugt werden Mittel, welche als Phosphonate 1-Hydroxyethan-1,1-diphosphonsäure (HEDP) oder Diethylentriaminpenta(methylenphosphonsäure) (DTPMP) enthalten. Selbstverständlich können die erfindungsgemäßen Mittel zwei oder mehr unterschiedliche Phosphonate enthalten. Bevorzugte erfindungsgemäße Mittel sind dadurch gekennzeichnet, dass das Mittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht des Mittels vorzugsweise 0,1 und 8,0 Gew.-%, bevorzugt 0,2 und 5,0 Gew.-% und insbesondere 0,5 und 3,0 Gew.-% beträgt.

Die erfindungsgemäßen Mittel enthalten weiterhin vorzugsweise Gerüststoff. Zu den Gerüststoffe zählen dabei insbesondere die Silikate und Carbonate.

Als organische Cobuilder sind insbesondere Polycarboxylate / Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben. Organische Cobuildersubstanzen können gewünschtenfalls in Mengen bis zu 40 Gew.-%, insbesondere bis zu 25 Gew.-% und vorzugsweise von 1 bis 8 Gew.-% enthalten sein.

Brauchbare organische Gerüstsubstanzen sind z.B. die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren und Carboxymethylinuline, monomere und polymere Aminopolycarbonsäuren, insbesondere Glycindiessigsäure, Methylglycindiessigsäure, Glutamindiessigsäure, Nitrilotriessigsäure (NTA), Iminodisuccinat wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendiamintetrakis(methylenphosphonsäure), Lysintetra(methylenphosphosäure) und 1-Hydroxyethan-1,1-diphosphonsäure, polymere Hydroxyverbindungen wie Dextrin sowie polymere (Poly)carbonsäuren, insbesondere durch Oxidation von Polysacchariden bzw. Dextrinen zugängliche Polycarboxylate, und/oder polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Derartige organische Buildersubstanzen können gewünschtenfalls in Mengen bis zu 50 Gew.-%, insbesondere bis zu 25 Gew.-% und vorzugsweise von 10 bis 20 Gew.-% enthalten sein.

Die freien Säuren besitzen neben ihrer Builderwirkung typischerweise auch die Eigenschaft einer Säuerungskomponente und dienen somit auch zur Einstellung eines niedrigeren und milderen pH-Wertes von Reinigungsmitteln. Insbesondere sind hierbei Citronensäure, Bernsteinsäure, Glutarsäure, Adipinsäure, Gluconsäure und beliebige Mischungen aus diesen zu nennen. Mit besonderem Vorzug wird als Gerüstsubstanz die Citronensäure oder Salze der Citronensäure eingesetzt. Weitere besonders bevorzugte Gerüstsubstanzen sind ausgewählt unter Methylglycindiessidsäure (MGDA), Glutaminsäurediacetat (GLDA), Asparaginsäurediacetat (ASDA), Hydroxyethyliminodiacetat (HEIDA), Iminodisuccinat (IDS) und Ethylendiamindisuccinat (EDDS), Carboxymethylinulin und Polyaspartat.

In bevorzugten Ausführungsformen wird als wasserlöslicher, organischer Builder Citronensäure und/oder Citrat eingesetzt. Besonders bevorzugt ist der Einsatz von 5 bis 25 Gew.-%, vorzugsweise 7,5 bis 12,5 Gew.-% Citronensäure und/oder 5 bis 25 Gew.-%, vorzugsweise 7,5 bis 12,5 Gew.-% Citrat, vorzugsweise Alkalicitrat, noch bevorzugter Natriumcitrat. Citronensäure/Citrat können jeweils in Form ihrer Hydrate eingesetzt werden, so kann z.B. Citronensäure in Form des Monohydrats, Citrat in Form des Trinatriumcitratdihydrats eingesetzt werden.

Als Gerüststoffe sind weiter polymere Polycarboxylate geeignet, dies sind z.B. die Alkalimetallsalze der Polyacrylsäure oder der Polymethacrylsäure, z.B. solche mit einer relativen Molekülmasse von 500 bis 70000 g/mol. Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im Allgemeinen 2000 bis 70000 g/mol, vorzugsweise 20000 bis 50000 g/mol und insbesondere 30000 bis 40000 g/mol.

In Ergänzung zu den zuvor beschriebenen Gerüststoffen können in dem Reinigungsmittel reinigungsaktive Polymere enthalten sein. Der Gewichtsanteil der reinigungsaktiven Polymere am Gesamtgewicht erfindungsgemäßer Reinigungsmittel beträgt vorzugsweise 0,1 bis 20 Gew.-%, vorzugsweise 1,0 bis 15 Gew.-% und insbesondere 2,0 bis 12 Gew.-%.

Als reinigungsaktive Polymere werden vorzugsweise Sulfonsäuregruppen-haltige Polymere, insbesondere aus der Gruppe der copolymeren Polysulfonate, eingesetzt. Diese copolymeren Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist. Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Bei den Sulfonsäuregruppen-haltigen Monomeren sind solche der Formel R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, - C(O)-NH-C(CH₃)₂-, -C(O)-NH-C(CH₃)₂-CH₂- und -C(O)-NH-CH(CH₂CH₃)-.

Unter diesen Monomeren bevorzugt sind solche der Formeln H₂C=CH-X-SO₃H, H₂C=C(CH₃)-X-SO₃H und HO₃S-X-(R⁶)C=C(R⁷)-X-SO₃H, in denen R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-,-C(O)-NH-C(CH₃)₂-CH₂- und -C(O)-NH-CH(CH₂CH₃)-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen. Der Einsatz von teil- oder vollneutralisierten sulfonsäuregruppenhaltigen Copolymeren ist erfindungsgemäß bevorzugt. Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte maschinelle Geschirrspülmittel sind dadurch gekennzeichnet, dass die Copolymere Molmassen von 2000 bis 200000 g/mol, vorzugsweise 4000 bis 25000 g/mol und insbesondere 5000 bis 15000 g/mol aufweisen.

In einer weiteren bevorzugten Ausführungsform umfassen die Copolymere neben Carboxylgruppen-haltigem Monomer und Sulfonsäuregruppen-haltigen Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

Geschirrspülmittel, enthaltend ein Copolymer, umfassend i) Carbonsäuregruppen-haltige Monomer(e), ii) Sulfonsäuregruppen-haltige Monomer(e), iii) nichtionische Monomer(e) werden erfindungsgemäß bevorzugt. Durch den Einsatz dieser Terpolymere konnte die Klarspülleistung erfindungsgemäßer maschineller Geschirrspülmittel gegenüber vergleichbaren Geschirrspülmitteln, die Sulfopolymere ohne Zusatz nichtionischer Monomere enthalten, verbessert werden.

Als nichtionische Monomere werden vorzugsweise Monomere der allgemeinen Formel R¹(R²)C=C(R³)-X-R⁴ eingesetzt, in der R¹ bis R³ unabhängig voneinander für -H, -CH₃ oder -C2H5 steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht. Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie z.B. 1-Decen, 1-Dodecen, 1-Hexadecen, 1-Oktadecen und C₂₂-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2,Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und *N*-(Behenyl)acrylamid oder deren Mischungen.

Der Gewichtsanteil der Sulfonsäuregruppen-haltigen Copolymere am Gesamtgewicht erfindungsgemäßer Mittel beträgt vorzugsweise 0,1 bis 15 Gew.-%, vorzugsweise 1,0 bis 12 Gew.-% und insbesondere 2,0 bis 10 Gew.-%.

Als einen weiteren Bestandteil können die erfindungsgemäßen Mittel ein organisches Lösungsmittel enthalten. Der Zusatz organischer Lösungsmittel wirkt sich vorteilhaft auf die Enzymstabilität und die Reinigungsleistung dieser Mittel aus. Bevorzugte organische Lösungsmittel stammen aus der Gruppe ein- oder mehrwertigen Alkohole, Alkanolamine oder Glykolether. Vorzugsweise werden die Lösungsmittel ausgewählt aus Ethanol, n- oder i-Propanol, Butanol, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Etheylenglykolmonon-butylether, Diethylenglykolmethylether, Di-ethylenglykolethylether, Propylenglykolmethyl-, -ethyl- oder -propylether, Dipropylenglykolmethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel. Der Gewichtsanteil dieser organischen Lösungsmittel am Gesamtgewicht erfindungsgemäßer Mittel beträgt vorzugsweise 0,1 bis 10 Gew.-%, bevorzugt 0,2 bis 8,0 Gew.-% und insbesondere 0,5 bis 5,0 Gew.-%. Ein besonders bevorzugtes und in Bezug auf die Stabilisierung der Mittel besonders wirksames organisches Lösungsmittel ist das Glycerin sowie das 1,2-Propylenglykol. Flüssige Mittel die mindestens ein Polyol, vorzugsweise aus der Gruppe Glycerin und 1,2-Propylenglykol enthalten, wobei der Gewichtsanteil des Polyols am Gesamtgewicht des Mittels vorzugsweise 0,1 und 10 Gew.-%, bevorzugt 0,2 und 8,0 Gew.-% und insbesondere 0,5 und 5,0 Gew.-% beträgt, werden erfindungsgemäß bevorzugt. Weitere bevorzugte organische Lösungsmittel sind die organischen Amine und Alkanolamine. Die erfindungsgemäßen Mittel enthalten diese Amine vorzugsweise in Mengen von 0,1 bis 10 Gew.-%, bevorzugt 0,2 bis 8,0 Gew.-% und insbesondere 0,5 bis 5,0 Gew.-%, jeweils bezogen auf ihr Gesamtgewicht. Ein besonders bevorzugtes Alkanolamin ist das Ethanolamin.

Ein weiterer bevorzugter Bestandteil der erfindungsgemäßen Reinigungsmittel ist ein Zuckeralkohol (Alditol). Die Gruppe der Alditole umfasst nichtcyclische Polyole der Formel HOCH₂[CH(OH)]ₙCH₂OH. Zu den Alditolen zählen z.B. Mannit (Mannitol), Isomalt, Lactit, Sorbit (Sorbitol) und Xylit (Xylitol), Threit, Erythrit und Arabit. Als in Bezug auf die Enzymstabilität besonders vorteilhaft hat sich das Sorbitol erwiesen. Der Gewichtsanteil des Zuckeralkohols am Gesamtgewicht des Reinigungsmittels beträgt vorzugsweise 1,0 bis 10 Gew.-%, bevorzugt 2,0 bis 8,0 Gew.-% und insbesondere 3,0 bis 6,0 Gew.-%.

Ein erfindungsgemäßes Mittel enthält die Protease vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels. Ferner kann die in dem Mittel enthaltene Protease, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Reinigungsmittel selbst in einem Behältnis, vorzugsweise einem luftdurchlässigen Behältnis, verpackt sein, aus dem es kurz vor Gebrauch oder während des Spülvorgangs freigesetzt wird.

Diese Ausführungsformen der vorliegenden Erfindung umfassen alle festen, pulverförmigen, granularen, tablettenförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 bis 1200 g/l, insbesondere 500 bis 900 g/l oder 600 bis 850 g/l. Zu den festen Darreichungsformen des Mittels zählen ferner Extrudate, Granulate, Tabletten oder Pouches enthaltend feste Mittel, die sowohl in Großgebinden als auch portionsweise abgepackt vorliegen können. Alternativ kann das Mittel auch flüssig, gelförmig oder pastös sein, z.B. in Form eines nicht-wässrigen Mittels oder einer nicht-wässrigen Paste oder in Form eines wässrigen Mittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen (Mehrkomponentensystem). Ein solches Mittel ist demnach in mehrere Komponenten aufgeteilt, z.B. zwei flüssige, zwei feste oder eine flüssige und eine feste Phase. Die flüssige Angebotsform auf Basis von Wasser und/oder organischen Lösungsmitteln können verdickt, in Form von Gelen vorliegen.

Ein Stoff, z.B. eine Zusammensetzung oder ein Mittel ist gemäß Definition der Erfindung festförmig, wenn sie bei 25°C und 1.013 mbar im festen Aggregatzustand vorliegt.

Ein Stoff, z.B. eine Zusammensetzung oder ein Mittel ist gemäß Definition der Erfindung flüssig, wenn sie bei 25°C und 1.013 mbar im flüssigen Aggregatzustand vorliegt. Dabei umfasst flüssig auch gelförmig.

Wenn die erfindungsgemäßen Mittel in flüssiger Form vorliegen, enthalten sie vorzugsweise, bezogen auf ihr Gesamtgewicht mehr als 40 Gew.-%, vorzugsweise zwischen 50 und 90 Gew.-% und insbesondere zwischen 60 und 80 Gew.-% Wasser.

Die hierin beschriebenen Mittel, insbesondere Geschirrspülmittel, noch bevorzugter maschinellen Geschirrspülmittel, werden vorzugsweise zu Dosiereinheiten vorkonfektioniert. Diese Dosiereinheiten umfassen vorzugsweise die für einen Reinigungsgang notwendige Menge an reinigungsaktiven Substanzen. Bevorzugte Dosiereinheiten weisen ein Gewicht zwischen 12 und 30 g, bevorzugt zwischen 14 und 26 g und insbesondere zwischen 15 und 22 g auf. Das Volumen der vorgenannten Dosiereinheiten sowie deren Raumform sind mit besonderem Vorzug so gewählt, dass eine Dosierbarkeit der vorkonfektionierten Einheiten über die Dosierkammer einer Geschirrspülmaschine gewährleistet ist. Das Volumen der Dosiereinheit beträgt daher bevorzugt zwischen 10 und 35 ml, vorzugsweise zwischen 12 und 30 ml.

Die Mittel, insbesondere Geschirrspülmittel, insbesondere die vorgefertigten Dosiereinheiten weisen mit besonderem Vorzug eine wasserlösliche Umhüllung auf. Die wasserlösliche Umhüllung wird vorzugsweise aus einem wasserlöslichen Folienmaterial, welches ausgewählt ist aus der Gruppe, bestehend aus Polymeren oder Polymergemischen, gebildet. Die Umhüllung kann aus einer oder aus zwei oder mehr Lagen aus dem wasserlöslichen Folienmaterial gebildet werden. Das wasserlösliche Folienmaterial der ersten Lage und der weiteren Lagen, falls vorhanden, kann gleich oder unterschiedlich sein. Besonders bevorzugt sind Folien, die z.B. zu Verpackungen wie Schläuchen oder Kissen verklebt und/oder versiegelt werden können, nachdem sie mit einem Mittel befüllt wurden.

Die wasserlösliche Verpackung kann eine oder mehr Kammern aufweisen. Das Mittel kann in einer oder mehreren Kammern, falls vorhanden, der wasserlöslichen Umhüllung enthalten sein. Die Menge an Mittel entspricht vorzugsweise der vollen oder halben Dosis, die für einen Spülgang benötigt wird.

Es ist bevorzugt, dass die wasserlösliche Umhüllung Polyvinylalkohol oder ein Polyvinylalkoholcopolymer enthält. Wasserlösliche Umhüllungen, die Polyvinylalkohol oder ein Polyvinylalkoholcopolymer enthalten, weisen eine gute Stabilität bei einer ausreichend hohen Wasserlöslichkeit, insbesondere Kaltwasserlöslichkeit, auf. Geeignete wasserlösliche Folien zur Herstellung der wasserlöslichen Umhüllung basieren bevorzugt auf einem Polyvinylalkohol oder einem Polyvinylalkoholcopolymer, dessen Molekulargewicht im Bereich von 5000 bis 1000000 g/mol, vorzugsweise von 20000 bis 500000 g/mol, besonders bevorzugt von 30000 bis 100000 g/mol und insbesondere von 40000 bis 80000 g/mol liegt. Geeignete wasserlösliche Folien zum Einsatz in den wasserlöslichen Umhüllungen der wasserlöslichen Verpackungen gemäß der Erfindung sind Folien, die von der Firma MonoSol LLC z.B. unter der Bezeichnung M8630, C8400 oder M8900 vertrieben werden. Andere geeignete Folien umfassen Folien mit der Bezeichnung Solublon® PT, Solublon® GA, Solublon® KC oder Solublon® KL von der Aicello Chemical Europe GmbH oder die Folien VF-HP von Kuraray.

Solche wasserlöslichen Umhüllungen sind auch in den Patentanmeldungen WO2004031338A sowie WO2003099985A, auf deren Offenbarung hiermit in vollem Umfang Bezug genommen wird, bereits beschrieben.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen z.B. die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt. Weiterhin ist es möglich, zwei oder mehrere Enzyme zusammen zu konfektionieren, so dass ein einzelnes Granulat mehrere Enzymaktivitäten aufweist.

Erfindungsgemäße Reinigungsmittel können ausschließlich eine *Bacillus gibsonii* Protease, wie hierin definiert, enthalten. Alternativ können sie auch weitere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder andere - von den erfindungsgemäßen Protease unterscheidbare - Protease, sowie deren Gemische. Weitere Enzyme sind in dem Mittel vorteilhafterweise jeweils in einer Menge von 1 x 10⁻⁸ bis 5 Gew.-% bezogen auf aktives Protein enthalten. Zunehmend bevorzugt ist jedes weitere Enzym in einer Menge von 1 x 10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels, darunter insbesondere zwischen der genannten Protease und einer Amylase und/oder einer Lipase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Beispiele für Proteasen sind die Subtilisine BPN' aus *Bacillus amyloliquefaciens* und Carlsberg aus *Bacillus licheniformis,* die Protease PB92, die Subtilisine 147 und 309, die Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von dem Unternehmen Novozymes erhältlich. Die Subtilisine 147 und 309 werden unter den Handelsnamen Esperase® bzw. Savinase® von dem Unternehmen Novozymes vertrieben. Von der Protease aus *Bacillus lentus* DSM 5483 leiten sich die unter der Bezeichnung BLAP® geführten Protease-Varianten ab. Weitere brauchbare Proteasen sind z.B. die unter den Handelsnamen Durazym®, Relase®, Everlase®, Nafizym®, Natalase®, Kannase® und Ovozyme® von dem Unternehmen Novozymes, die unter den Handelsnamen, Purafect®, Purafect® OxP, Purafect® Prime, Excellase® und Properase® von dem Unternehmen Danisco/Genencor, das unter dem Handelsnamen Protosol® von dem Unternehmen Advanced Biochemicals Ltd., das unter dem Handelsnamen Wuxi® von dem Unternehmen Wuxi Snyder Bioproducts Ltd., die unter den Handelsnamen Proleather® und Protease P® von dem Unternehmen Amano Pharmaceuticals Ltd., und das unter der Bezeichnung Proteinase K-16 von dem Unternehmen Kao Corp. erhältlichen Enzyme. Besonders bevorzugt eingesetzt werden auch die Proteasen aus *Bacillus gibsonii* und *Bacillus pumilus,* die offenbart sind in den internationalen Patentanmeldungen WO2008086916 und WO2007131656. Weitere vorteilhaft einsetzbare Proteasen sind offenbart in den Patentanmeldungen WO9102792, WO2008007319, WO9318140, WO0144452, GB1243784, WO9634946, WO2002029024 und WO2003057246. Weitere verwendbare Proteasen sind diejenigen, die in den Mikroorganismen *Stenotrophomonas maltophilia,* insbesondere *Stenotrophomonas maltophilia* K279a, *Bacillus intermedius* sowie *Bacillus sphaericus* natürlicherweise vorhanden sind.

Beispiele für Amylasen sind die α-Amylasen aus *Bacillus licheniformis, Bacillus amyloliquefaciens* oder *Bacillus stearothermophilus* sowie insbesondere auch deren für den Einsatz in Reinigungsmitteln verbesserte Weiterentwicklungen. Das Enzym aus *Bacillus licheniformis* ist von dem Unternehmen Novozymes unter dem Namen Termamyl® und von dem Unternehmen Danisco/Genencor unter dem Namen Purastar® ST erhältlich. Weiterentwicklungsprodukte dieser α-Amylase sind von dem Unternehmen Novozymes unter den Handelsnamen Duramyl® und Termamyl® ultra, von dem Unternehmen Danisco/Genencor unter dem Namen Purastar® OxAm und von dem Unternehmen Daiwa Seiko Inc. als Keistase® erhältlich. Die α-Amylase von *Bacillus amyloliquefaciens* wird von dem Unternehmen Novozymes unter dem Namen BAN® vertrieben, und abgeleitete Varianten von der α-Amylase aus *Bacillus stearothermophilus* unter den Namen BSG® und Novamyl®, ebenfalls von dem Unternehmen Novozymes. Des Weiteren sind für diesen Zweck die α-Amylase aus *Bacillus sp.* A 7- 7 (DSM 12368) und die Cyclodextrin-Glucanotransferase (CGTase) aus *Bacillus agaradherens* (DSM 9948) hervorzuheben. Ferner sind die amylolytischen Enzyme einsetzbar, die in den internationalen Patentanmeldungen WO2003002711, WO2003054177 und WO2007079938 offenbart sind, auf deren Offenbarung daher ausdrücklich verwiesen wird bzw. deren diesbezüglicher Offenbarungsgehalt daher ausdrücklich in die vorliegende Patentanmeldung mit einbezogen wird. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus *Aspergillus niger* und *A. oryzae* geeignet. Weitere vorteilhaft einsetzbare Handelsprodukte sind z.B. die Amylase-LT® und Stainzyme® oder Stainzyme ultra® bzw. Stainzyme plus® sowie Amplify™ oder Amplify Prime™, ebenfalls von dem Unternehmen Novozymes. Auch durch Punktmutationen erhältliche Varianten dieser Enzyme können erfindungsgemäß eingesetzt werden.

Beispiele für Lipasen oder Cutinasen, die insbesondere wegen ihrer Triglycerid-spaltenden Aktivitäten, aber auch, um aus geeigneten Vorstufen *in situ* Persäuren zu erzeugen, eingesetzt werden, umfassen z.B. die ursprünglich aus *Humicola lanuginosa (Thermomyces lanuginosus)* erhältlichen bzw. daraus weiterentwickelten Lipasen, insbesondere solche mit einem oder mehreren der folgenden Aminosäureaustausche ausgehend von der genannten Lipase in den Positionen D96L, T213R und/oder N233R, besonders bevorzugt T213R und N233R. Lipasen werden z.B. von dem Unternehmen Novozymes unter den Handelsnamen Lipolase®, Lipolase® Ultra, LipoPrime®, Lipozyme® und Lipex® vertrieben. Eine weitere vorteilhaft einsetzbare Lipase ist unter dem Handelsnamen Lipoclean® von dem Unternehmen Novozymes erhältlich. Des Weiteren sind z.B. die Cutinasen einsetzbar, die ursprünglich aus *Fusarium solani pisi* und *Humicola insolens* isoliert worden sind. Ebenso brauchbare Lipasen sind von dem Unternehmen Amano unter den Bezeichnungen Lipase CE®, Lipase P®, Lipase B®, bzw. Lipase CES®, Lipase AKG®, Bacillus sp. Lipase®, Lipase AP®, Lipase M-AP® und Lipase AML® erhältlich. Von dem Unternehmen Danisco/Genencor sind z.B. die Lipasen bzw. Cutinasen einsetzbar, deren Ausgangsenzyme ursprünglich aus *Pseudomonas mendocina* und *Fusarium solanii* isoliert worden sind. Als weitere wichtige Handelsprodukte sind die ursprünglich von dem Unternehmen Gist-Brocades (inzwischen Danisco/Genencor) vertriebenen Präparationen M1 Lipase® und Lipomax® und die von dem Unternehmen Meito Sangyo KK unter den Namen Lipase MY-30®, Lipase OF® und Lipase PL® vertriebenen Enzyme zu erwähnen, ferner das Produkt Lumafast® von dem Unternehmen Danisco/Genencor.

Zur Erhöhung der bleichenden Wirkung können erfindungsgemäß Oxidoreduktasen, z.B. Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Ein weiterer Erfindungsgegenstand ist ein Verfahren zur Reinigung von harten Oberflächen, insbesondere Geschirr, dadurch gekennzeichnet, dass in mindestens einem Verfahrensschritt ein erfindungsgemäßes Mittel angewendet wird.

Ein bevorzugtes Reinigungsverfahren ist ein maschinelles Geschirrspülverfahren. Die Dosierung des erfindungsgemäßen Mittels in die Reinigungsflotte kann in einem solchen Verfahren z.B. mittels der Dosierkammer in der Tür oder mittels eines zusätzlichen Dosierbehälters im Innenraum der Geschirrspülmaschine erfolgen. Alternativ kann das Mittel auch direkt auf das verschmutzte Geschirr oder auf eine der Innenwände der Geschirrspülmaschine, z.B. die Innenseite der Tür aufgebracht werden. Die Durchführung des erfindungsgemäßen Verfahrens erfolgt im Innenraum einer handelsüblichen Geschirrspülmaschine. Das Reinigungsprogramm kann bei einer Geschirrspülmaschine in der Regel vor Durchführung des Geschirrspülverfahrens durch den Verbraucher gewählt und festgelegt werden. Das in dem erfindungsgemäßen Verfahren eingesetzte Reinigungsprogramm der Geschirrspülmaschine umfasst dabei mindestens einen Vorspülgang und einen Reinigungsgang. Erfindungsgemäß bevorzugt werden Reinigungsprogramme, die weitere Reinigungs- oder Spülgänge, z.B. einen Klarspülgang umfassen. Das erfindungsgemäße Verfahren ist mit besonderem Vorzug Bestandteil eines Reinigungsprogramms, umfassend einen Vorspülgang, einen Reinigungsgang sowie einen Klarspülgang. Das erfindungsgemäße Verfahren wird bevorzugt in Verbindung mit solchen Reinigungsprogrammen eingesetzt, bei denen die Waschflotte im Verlauf des Reinigungsgangs erwärmt wird. In einer bevorzugten Ausführungsform des erfindungsgemäßen Verfahrens ist der Reinigungsgang, in dessen Verlauf das erfindungsgemäße Mittel in den Innenraum der Geschirrspülmaschine eindosiert wird dadurch gekennzeichnet, dass in seinem Verlauf die Temperatur der Reinigungsflotte auf Werte oberhalb 30°C, vorzugsweise oberhalb 40°C und insbesondere oberhalb 50°C ansteigt.

In verschiedenen Ausführungsformen zeichnet sich das oben beschriebene Verfahren dadurch aus, dass die Protease bei einer Temperatur von 0 bis 100°C, bevorzugt 10 bis 70°C, weiter bevorzugt 30 bis 50°C und am meisten bevorzugt bei 45°C eingesetzt wird.

Alle Sachverhalte, Gegenstände und Ausführungsformen, die für erfindungsgemäße Protease und sie enthaltende Mittel beschrieben sind, sind auch auf die Erfindungsgegenstände, die entsprechende Verfahren und Verwendungen betreffen anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

### Ermittlung der Reinigungsleistung erfindungsgemäßer Geschirrspülmittel

Es wurde die Reinigungsleistung eines handelsüblichen automatischen Geschirrspülmittels in Form einer Geschirrspülmitteltablette, das 15 Gew.-% Natriumpercarbonat als Wasserstoffperoxidquelle (Bleichmittel), 0,03 Gew.-% Mn-Me-TACN (Mn-TACN) als Bleichkatalysator und 2,2 Gew.-% TAED als Bleichaktivator enthielt, getestet, dem jeweils Proteasegranulat 1 oder 2 zugesetzt wurde. Die Proteasen wurden jeweils aktivproteingleich eingesetzt.

Die Reinigungsleistung wurde gemäße IKW Methode in einer Spülmaschine Miele GSL (Programm: 45°C, Wasserhärte 21°dH) bestimmt. Die Geschirrspülmitteltablette wurde vor Beginn des Reinigungsprogramms in die Dosiervorrichtung gegeben. Es wurden jeweils drei Bestimmungen durchgeführt, deren Ergebnisse jeweils gemittelt wurden. Die Auswertung der Reinigungsleistung erfolgte visuell gemäß einer Skala von 1 bis 10, wobei der Wert 10 die beste Note ist (kein erkennbarer Rückstand). Werte werden dann als signifikant angesehen, wenn die Verbesserung mindestens 1,0 beträgt. Die Ergebnisse sind in den nachfolgenden Tabellen dargestellt.

**Tabelle 1:**

| | | Eingebranntes Hackfleisch | Eigelb | Tee (Assam) | Tee (BOP) |
|---|---|---|---|---|---|
| E1 | Geschirrspülmittel mit Proteasegranulat 1 (30 mg/job) | 6,5 | 5,2 | 6,8 | 7,5 |
| E2 | Geschirrspülmittel mit Proteasegranulat 2 (30 mg/job) | 5,2 | 4,1 | 6,0 | 5,9 |

**Tabelle 2:**

| | | Creme Brûlée | Eigelb | Tee (Assam) | Tee (BOP) |
|---|---|---|---|---|---|
| E3 | Geschirrspülmittel mit Proteasegranulat 1 (120 mg/job) | 9,1 | 8,4 | 7,3 | 7,5 |
| E4 | Geschirrspülmittel mit Proteasegranulat 2 (120 mg/job) | 7,7 | 6,9 | 6,3 | 6,7 |

**Tabelle 3:**

| | | Creme Brûlée | Tee (Assam) |
|---|---|---|---|
| E5 | Geschirrspülmittel mit Proteasegranulat 1 (220 mg/job) | 9,7 | 7,6 |
| E6 | Geschirrspülmittel mit Proteasegranulat 2 (220 mg/job) | 8,6 | 6,3 |

Proteasegranulat 1 enthält eine Protease gemäß SEQ ID NO:5 in WO2017215925 beschrieben Proteasegranulat 2 enthält eine Kombination aus Protease 2a (Protease gemäß SEQ ID NO:6 aus WO2016000973) und Protease 2b (Ovozyme®, ex Novozymes) im Verhältnis 1,5:1.

Überraschenderweise wurde gefunden, dass bei Einsatz von Proteasegranulat 1 neben einer signifikant verbesserten Reinigungsleistung auf proteinhaltige Anschmutzungen auch eine signifikant verbesserte Reinigungsleistung an Teeanschmutzungen zu beobachten ist.

## Patentansprüche

1. Geschirrspülmittel, umfassend eine Wasserstoffperoxidquelle, einen Bleichkatalysator und eine Protease, **dadurch gekennzeichnet, dass** die Protease eine Aminosäuresequenz umfasst, die mindestens 70% Sequenzidentität mit der in SEQ ID NO:1 angegebenen Aminosäuresequenz über deren Gesamtlänge aufweist und mindestens eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist.

2. Geschirrspülmittel nach Anspruch 1, **dadurch gekennzeichnet, dass** der Bleichkatalysator ausgewählt ist aus der Gruppe der bleichverstärkenden Übergangsmetallsalze und Übergangsmetallkomplexe, vorzugsweise aus der Gruppe der Komplexe des Mangans mit 1,4,7-trimethyl-1,4,7-triazacyclononan (Me-TACN) oder 1,2, 4,7-tetramethyl-1,4,7-triazacyclononan (Me/Me-TACN), und/oder die Wasserstoffperoxidquelle Natriumpercarbonat, Natriumperborattetrahydrat oder Natriumperboratmonohydrat oder eine Kombination hiervon ist.

3. Geschirrspülmittel nach Anspruch 1 oder 2, **dadurch gekennzeichnet, dass** es die Protease in einer Menge von 1 x 10⁻⁸ bis 10 Gew.-%, bezogen auf aktives Protein, enthält, und/oder den Bleichkatalysator in einer Menge von 0,0025 bis 1 Gew.-% enthält, und/oder die Wasserstoffperoxidquelle in einer Menge von 2 bis 30 Gew.-% enthält.

4. Geschirrspülmittel nach einem der Ansprüche 1 bis 3, **dadurch gekennzeichnet, dass** es ferner einen Bleichaktivator umfasst, insbesondere TAED, vorzugsweise in einer Menge von 0,1 bis 10 Gew.-%.

5. Geschirrspülmittel nach einem der Ansprüche 1 bis 4, **dadurch gekennzeichnet, dass** die Protease eine Aminosäuresequenz, die mindestens 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% Sequenzidentität mit der in SEQ ID NO:1 angegebenen Aminosäuresequenz über deren Gesamtlänge aufweist und eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist, wobei die mindestens eine Aminosäuresubstitution aus der aus Q12L, I43V, M122L, D127P, N154S, T156A, G160S, M211N, M211L, P212D, P212H oder A222S bestehenden Gruppe ausgewählt ist.

6. Geschirrspülmittel nach einem der Ansprüche 1 bis 5, **dadurch gekennzeichnet, dass** die Protease eine der folgenden Aminosäuresubstitutionsvarianten umfasst: (i) I43V; (ii) M122L, N154S und T156A; (iii) M211N und P212D; (iv) M211L und P212D; (v) G160S; (vi) D127P, M211L und P212D; (vii) P212H; oder (viii) Q12L, M122L und A222S, wobei die Nummerierung jeweils bezogen ist auf die Nummerierung gemäß SEQ ID NO:1.

7. Geschirrspülmittel nach einem der Ansprüche 1 bis 6, **dadurch gekennzeichnet, dass** es sich um ein maschinelles Geschirrspülmittel handelt.

8. Geschirrspülmittel nach einem der Ansprüche 1 bis 7, **dadurch gekennzeichnet, dass** es in fester Form vorliegt, insbesondere als rieselfähiges Pulver oder als Formkörper, oder dass es in flüssiger, gelförmiger oder pastöser Form vorliegt.

9. Geschirrspülmittel nach einem der Ansprüche 1 bis 8, **dadurch gekennzeichnet, dass** es mindestens einen weiteren Inhaltsstoff umfasst, der ausgewählt ist aus der Gruppe bestehend aus Gerüststoff, Tensid, anionisches Polymer sowie Kombinationen hiervon, und/oder dass es mindestens ein weiteres Enzym umfasst, insbesondere eine Protease, Amylase, Cellulase, Pektin-spaltendes Enzym, Hemicellulase, Mannanase, Tannase, Xylanase, Xanthanase, ß-Glucosidase, Carrageenase, Perhydrolase, Oxidase, Oxidoreduktase oder eine Lipase, sowie Kombinationen hiervon, insbesondere eine Kombination, die ausgewählt ist aus Protease und Amylase, Protease und Lipase, Protease und Cellulase, Protease und Mannanase, Amylase und Lipase, Amylase und Cellulase, Amylase und Mannanase, Lipase und Cellulase, Lipase und Mannanase, Lipase und Cellulase, Protease und Amylase und Lipase, Protease und Amylase und Cellulase, Protease und Amylase und Mannanase, Amylase und Lipase und Cellulase, Amylase und Lipase und Mannanase, Lipase, Cellulase und Mannanase, Protease und Amylase und Lipase und Cellulase, Protease und Amylase und Cellulase und Mannanase.

10. Verfahren zur Entfernung von proteinhaltigen Anschmutzungen, insbesondere Eianschmutzungen, und/oder bleichbaren Anschmutzungen, insbesondere Teeanschmutzungen, auf harten Oberflächen, insbesondere Geschirr, umfassend einen der Verfahrensschritte
(a) In Kontakt bringen der harten Oberfläche mit einer Reinigungsflotte, die ein Geschirrspülmittel gemäß einem der Ansprüche 1 bis 9 enthält, oder
(b) In Kontakt bringen der harten Oberfläche mit einer Reinigungsflotte, die eine Wasserstoffperoxidquelle, einen Bleichkatalysator und eine Protease, die eine Aminosäuresequenz umfasst, die mindestens 70% Sequenzidentität mit der in SEQ ID NO:1 angegebenen Aminosäuresequenz über deren Gesamtlänge aufweist und mindestens eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist.

11. Verwendung eines Geschirrspülmittels nach einem der Ansprüche 1 bis 9 zur Entfernung von proteinhaltigen Anschmutzungen, insbesondere Eianschmutzungen, und/oder bleichbaren Anschmutzungen, insbesondere Teeanschmutzungen, auf harten Oberflächen.

12. Verwendung einer Protease, die eine Aminosäuresequenz umfasst, die mindestens 70% Sequenzidentität mit der in SEQ ID NO:1 angegebenen Aminosäuresequenz über deren Gesamtlänge aufweist und mindestens eine Aminosäuresubstitution an mindestens einer der Positionen, die den Positionen 12, 43, 122, 127, 154, 156, 160, 211, 212 und 222, jeweils bezogen auf die Nummerierung gemäß SEQ ID NO:1, entsprechen, aufweist, zur Entfernung von proteinhaltigen Anschmutzungen, insbesondere Eianschmutzungen, und/oder bleichbaren Anschmutzungen, insbesondere Teeanschmutzungen, auf harten Oberflächen in einer Reinigungsflotte, die ferner eine Wasserstoffperoxidquelle und einen Bleichkatalysator umfasst.
